# NEW EUROPEAN PATENT SPECIFICATION

(11) **EP 2 655 623 B2**
(45) Date of publication and mention of the opposition decision: **30.08.2023**
(45) Mention of the grant of the patent: 22.02.2017
(21) Application number: 11805869.2
(22) Date of filing: 23.12.2011
(51) Int. Cl.: C12N 15/117

(54) **NON-CODING IMMUNOMODULATORY DNA CONSTRUCT**
NICHT-CODIERENDE IMMUNMODULATORISCHE DNA KONSTRUKTE
MOLECULE D'ACIDE NUCLEIQUE NON CODANTS IMMUNOMODULATEURS

(30) Priority: 23.12.2010 GB 201021867
(43) Date of publication of application: 30.10.2013
(73) Proprietor: Gilead Sciences, Inc., Foster City, CA 94404 (US)
(72) Inventor: SCHROFF, Matthias, 14195 Berlin (DE); KLEUSS, Christiane, 12203 Berlin (DE); KAPP, Kerstin, 10247 Berlin (DE)
(74) Representative: Carpmaels & Ransford LLP
(86) International application number: PCT/EP2011/074033
(87) International publication number: WO 2012/085291

(56) References cited:
- EP-A1- 2 246 433
- EP-A2- 2 333 091
- EP-B1- 1 196 178
- EP-B1- 2 197 488
- WO-A1-2012/085291
- WO-A2-02/26757
- WO-A2-03/057822
- WO-A2-2006/002038
- WO-A2-2007/047396
- DE-A1- 10 211 558
- DE-A1- 10 211 558
- US-A1- 2002 095 033
- US-A1- 2008 260 820
- US-A1- 2009 169 472
- US-B1- 6 251 666
- US-B2- 7 879 992
- A. D. MCNAUGHT et al.: "Definition of ''Conformation'' in IUPAC . Compendium of Chemical Terminology", Gold Book, 1997, Oxford ISBN: 0-9678550-9-8 Retrieved from the Internet: URL:http://goldbook.iupac.org
- A. D. MCNAUGHT et al.: "Configuration (stereo chemical)", IUPAC. Compendium of Chemical Terminology Gold Book, 1997, Oxford ISBN: 0-9678550-9-8 Retrieved from the Internet: URL:http://goldbook.iupac.org
- TONDELLI et al.: "Terminally L? modified oligonucleotides: pairing, stability and biological properties", Anti-Cancer Drugs, vol. 7, February 1996 (1996-02), pages 189-194,
- KIM et al.: "Superior structure stability and selectivity of hairpin nucleic acid probes with an L-DNA stem", Nucleic Acid Res., vol. 35, no. 21, 2007, pages 7279-7287,
- URATA et al.: "NMR study of a heterochiral DNA: stable Watson-Crick-type base-pairing between the enantiomeric residues", J. Am. Chem. Soc., vol. 115, 1993, pages 9852-9853,
- GARBESI et al.: "L-DNAs as potential antimessenger oligonucleotides: a reassessment", Nucleic Acids Res., vol. 21, no. 18, 11 September 1993 (1993-09-11), pages 4159-4165,
- SOSUNOV et al.: "Stereochemical control of DNA biosynthesis", Nucleic Acids Res., vol. 28, no. 5, 1 March 2000 (2000-03-01), pages 1170-117,
- HAYASHI et al.: "Application of L-DNA as a molecular tag", Nucleic Acids Symp. Series., vol. 49, 2005, pages 261-262,
- HAUSER et al.: "Utilising the left-helical conformation of L-DNA for analysing different marker types on a single universal microarray platform", Nucleic Acids Res., vol. 34, no. 18, 2006, pages 5101-5111,
- HAYASHI et al.: "Detection ofL-DNA-Tagged PCR Products by Surface Plasmon Resonance Imaging", ChemBioChem., vol. 8, no. 2, 22 January 2007 (2007-01-22), pages 169-171,
- DOI et al.: "Structural characteristics of enantiomorphic DNA: crystal analysis of racemates of the d(CGCGCG) duplex", J. Am. Chem. Soc., vol. 115, November 1993 (1993-11), pages 10432-10433,
- WILLIAMS et al.: "Bioactive and nuclease-resistant L-DNA ligand of vasopressin", PNAS, vol. 94, no. 21, 1997, pages 11285-11290,
- LEVA et al.: "GnRH binding RNA and DNA Spiegelmers: A novel approach toward GnRH antagonism", Chemistry & Biology, vol. 9, March 2002 (2002-03), pages 351-359,
- WLOTZKA et al.: "In vivo Properties of an Anti-GnRH Spiegelmer: An Example of an Oligonucleotide-Based Therapeutic Substance Class", PNAS, vol. 99, no. 13, 25 June 2002 (2002-06-25) , pages 8898-8902,
- PURSCHKE et al.: "A DNA Spiegelmer to staphylococcal enterotoxin B", Nucleic Acids Res., vol. 31, no. 12, 15 June 2003 (2003-06-15) , pages 3027-3032,
- HAYASHI et al.: "Genotyping by allele-specific l-DNA-tagged PCR", J. Biotechnol., vol. 135, no. 2, 1 June 2008 (2008-06-01), pages 157-160,
- GOODCHILD J: "Conjugates of oligonucleotides and modified oligonucleotides: a review of their synthesis and properties", Bioconjugate Chemistry, vol. 1, no. 3, 1990, pages 165-187,
- SING Y et al.: "Recent developments in oligonucleotide conjugation", Chem Soc. Rev., vol. 39, no. 6, 2010, pages 2054-2070,
- KRISHNAMACHARI Y et al.: "Innovative strategies for co-delivering antigens and CpG oligonucleotides", Advanced Drug Delivery Reviews, vol. 61, no. 3, 28 March 2009 (2009-03-28) , pages 205-217,
- KRIEG et al.: "Therapeutic potential of Toll-like receptor 9 activation", Nat. Rev. Drug Discov, vol. 5, 2006, pages 471-484,
- KRIEG et al.: "Development of TLR9 agonists for cancer therapy", J. Clin. Invest, vol. 117, no. 5, 2007, pages 1184-1194,
- KRIEG et al.: "CpG MOTIFS IN BACTERIAL DNA TRIGGER DIRECT B-CELL ACTIVATION", Nature, vol. 374, 6 April 1995 (1995-04-06), pages 546-549,
- Wikibooks entry "Structural Biochemistry / Organic Chemistry, Carbohydrates; obtained on April 30, 2018 (https://en.wikibooks.org/wiki/Structural_ Biochemistry/Organic_Chemistry/Carbohydrat es)
- M. J. J. Blommers et al. "Effects of the Introduction of L-Nucleotides into DNA. Solution Structure of the Heterochiral Duplex d(G-C-G-(L)T-G-C-G)-d(C-G-C A-C-G-C) Studied by NMR Spectroscopy",Biochemistry, 1994, 33, pages 7886 - 7896
- Axel Vater et al"Turning mirror-image oligonucleotides into drugs: the evolution of Spiegelmer® therapeutics", Drug Discovery Today, 2015,Volume 20, Number 1, pages 147-155
- Communication under R. 71(3) EPC of November 21,2016, together with the printing copy (?Druckexemplar") of the Opposed Patent
- Experimental report
- Description of the Opposed Patent as originally filed
- Urata et al., ..Synthesis and properties of mirror-image DNA?, 1992,Nucleic Acids Research. Vol. 20. No. 13. po. 3325 - 3332

### SEQUENCE LISTING

<110> MOLOGEN AG
<120> Non-coding immunomodulatory DNA construct
<130> 80523WO
<160> 52
<170> PatentIn version 3.5
<210> 1
   <211> 30
   <212> DNA
   <213> Artificial Sequence
<220>
   <223> synthetic oligodeoxynucleotide, lin 30L2
<400> 1
   tcattggaaa acgttcttcg gggcgttctt 30
<210> 2
   <211> 27
   <212> DNA
   <213> Artificial Sequence
<220>
   <223> synthetic oligodeoxynucleotide, CKm336
<220>
   <221> misc_feature
   <223> completely in L-conformation, except for the last T
<400> 2
   tggaaaacgt tcttcggggc gttcttt 27
<210> 3
   <211> 31
   <212> DNA
   <213> Artificial Sequence
<220>
   <223> synthetic oligodeoxynucleotide, CKm337
<220>
   <221> misc_feature
   <223> 1, 2, 29 and 30 in L-conformation
<400> 3
   tcattggaaa acgttcttcg gggcgttctt t 31
<210> 4
   <211> 31
   <212> DNA
   <213> Artificial Sequence
<220>
   <223> synthetic oligodeoxynucleotide, CKm338
<220>
   <221> misc_feature
   <223> all but the last phosphodiester bonds modified to phosphothioates
<400> 4
   tcattggaaa acgttcttcg gggcgttctt t 31
<210> 5
   <211> 31
   <212> DNA
   <213> Artificial Sequence
<220>
   <223> synthetic oligodeoxynucleotide, CKm339
<220>
   <221> misc_feature
   <223> first two as well as second- and third-to-last phosphodiester bonds modified to phosphothioates
<400> 5
   tcattggaaa acgttcttcg gggcgttctt t 31
<210> 6
   <211> 21
   <212> DNA
   <213> Artificial Sequence
<220>
   <223> synthetic oligodeoxynucleotide, ODN2216
<220>
   <221> misc_feature
   <223> all phosphodiester bonds modified to phosphothioates
<400> 6
   gggggacgat cgtcgggggg t 21
<210> 7
   <211> 25
   <212> DNA
   <213> Artificial Sequence
<220>
   <223> synthetic oligodeoxynucleotide, CKm508
<220>
   <221> misc_feature
   <223> Nucleotides 1-2 and 23-24: in L-conformation Nucleotides 3-7 and 17-22: G-stretch Nucleotides 9-10, 13-14 and 16-17: CG-motif
<400> 7
   gggggggacg atcgtcgggg ggggt 25
<210> 8
   <211> 21
   <212> DNA
   <213> Artificial Sequence
<220>
   <223> synthetic oligodeoxynucleotide, CKm458
<220>
   <221> misc_feature
   <223> Nucleotides 1-2 and 19-20: in L-conformation Nucleotides 4-5 and 15-18: G-stretch Nucleotides 7-8, 11-12 and 14-15: CG-motif
<400> 8
   ggaggacgat cgtcgggggg t 21
<210> 9
   <211> 20
   <212> DNA
   <213> Artificial Sequence
<220>
   <223> synthetic oligodeoxynucleotide, CKm481
<220>
   <221> misc_feature
   <223> Nucleotides 1-2 and 18-19: in L-conformation Nucleotides 3-5 and 15-17: G-stretch Nucleotides 7-8, 11-12 and 14-15: CG-motif
<400> 9
   gggggacgat cgtcgggggt 20
<210> 10
   <211> 21
   <212> DNA
   <213> Artificial Sequence
<220>
   <223> synthetic oligodeoxynucleotide, CKm461
<220>
   <221> misc_feature
   <223> Nucleotides 1-2 and 19-20: in L-conformation Nucleotides 5 and 15-18: G-stretch Nucleotides 7-8, 11-12 and 14-15: CG-motif
<400> 10
   ggaagacgat cgtcgggggg t 21
<210> 11
   <211> 21
   <212> DNA
   <213> Artificial Sequence
<220>
   <223> synthetic oligodeoxynucleotide, CKm361-2
<220>
   <221> misc_feature
   <223> Nucleotides 1-2 and 19-20: in L-conformation Nucleotides 3-5 and 15-18: G-stretch Nucleotides 7-8, 11-12 and 14-15: CG-motif
<400> 11
   gggggacgat cgtcgggggg t 21
<210> 12
   <211> 22
   <212> DNA
   <213> Artificial Sequence
<220>
   <223> synthetic oligodeoxynucleotide, CKm479
<220>
   <221> misc_feature
   <223> Nucleotides 1-2 and 20-21: in L-conformation Nucleotides 3-6 and 16-19: G-stretch Nucleotides 8-9, 12-13 and 15-16: CG-motif
<400> 12
   ggggggacga tcgtcggggg gt 22
<210> 13
   <211> 25
   <212> DNA
   <213> Artificial Sequence
<220>
   <223> synthetic oligodeoxynucleotide, CKm503
<220>
   <221> misc_feature
   <223> Nucleotides 1-2 and 23-24: in L-conformation Nucleotides 3-7 and 17-22: G-stretch Nucleotides 9-10, 13-14 and 16-17: CG-motif
<400> 13
   aagggggacg atcgtcgggg ggaat 25
<210> 14
   <211> 25
   <212> DNA
   <213> Artificial Sequence
<220>
   <223> synthetic oligodeoxynucleotide, CKm507
<220>
   <221> misc_feature
   <223> Nucleotides 1-2 and 23-24: in L-conformation Nucleotides 3-7 and 17-22: G-stretch Nucleotides 9-10, 13-14 and 16-17: CG-motif
<400> 14
   ttgggggacg atcgtcgggg ggttt 25
<210> 15
   <211> 21
   <212> DNA
   <213> Artificial Sequence
<220>
   <223> synthetic oligodeoxynucleotide, CKm459
<220>
   <221> misc_feature
   <223> Nucleotides 1-2 and 19-20: in L-conformation Nucleotides 3-5, 15-16 and 18: G-stretch Nucleotides 7-8, 11-12 and 14-15: CG-motif
<400> 15
   gggggacgat cgtcggaggg t 21
<210> 16
   <211> 22
   <212> DNA
   <213> Artificial Sequence
<220>
   <223> synthetic oligodeoxynucleotide, CKm506
<220>
   <221> misc_feature
   <223> Nucleotides 1-2 and 20-21: in L-conformation Nucleotides 3-5 and 16-19: G-stretch Nucleotides 7-8, 11-12 and 15-16: CG-motif
<400> 16
   gggggacgat cgtgcggggg gt 22
<210> 17
   <211> 21
   <212> DNA
   <213> Artificial Sequence
<220>
   <223> synthetic oligodeoxynucleotide, CKm478
<220>
   <221> misc_feature
   <223> Nucleotides 1-2 and 19-20: in L-conformation Nucleotides 3-5 and 15-16: G-stretch Nucleotides 7-8, 11-12 and 14-15: CG-motif
<400> 17
   ggggggcgat cgtcggaagg t 21
<210> 18
   <211> 21
   <212> DNA
   <213> Artificial Sequence
<220>
   <223> synthetic oligodeoxynucleotide, CKm480
<220>
   <221> misc_feature
   <223> Nucleotides 1-2 and 19-20: in L-conformation Nucleotides 3-5 and 15-18: G-stretch Nucleotides 7-8 and 14-15: CG-motif
<400> 18
   gggggacgat gctcgggggg t 21
<210> 19
   <211> 21
   <212> DNA
   <213> Artificial Sequence
<220>
   <223> synthetic oligodeoxynucleotide, CKm462
<220>
   <221> misc_feature
   <223> Nucleotides 1-2 and 19-20: in L-conformation Nucleotides 3-5 and 15-16: G-stretch Nucleotides 7-8, 11-12 and 14-15: CG-motif
<400> 19
   gggggacgat cgtcggaagg t 21
<210> 20
   <211> 21
   <212> DNA
   <213> Artificial Sequence
<220>
   <223> synthetic oligodeoxynucleotide, CKm505
<220>
   <221> misc_feature
   <223> Nucleotides 1-2 and 19-20: in L-conformation Nucleotides 3-5 and 16-18 : G-stretch Nucleotides 7-8, 11-12 and 15-16: CG-motif
<400> 20
   gggggacgat cgtgcggggg t 21
<210> 21
   <211> 22
   <212> DNA
   <213> Artificial Sequence
<220>
   <223> synthetic oligodeoxynucleotide, CKm476
<220>
   <221> misc_feature
   <223> Nucleotides 1-2 and 20-21: in L-conformation Nucleotides 3-5 and 16-17 : G-stretch Nucleotides 8-9, 12-13 and 15-16: CG-motif
<400> 21
   gggggaacga tcgtcggaag gt 22
<210> 22
   <211> 21
   <212> DNA
   <213> Artificial Sequence
<220>
   <223> synthetic oligodeoxynucleotide, CKm464
<220>
   <221> misc_feature
   <223> Nucleotides 1-2 and 19-20: in L-conformation Nucleotides 15-18: G-stretch Nucleotides 7-8, 11-12 and 14-15: CG-motif
<400> 22
   ggcccccgat cgtcgggggg t 21
<210> 23
   <211> 22
   <212> DNA
   <213> Artificial Sequence
<220>
   <223> synthetic oligodeoxynucleotide, CKm476
<220>
   <221> misc_feature
   <223> Nucleotides 1-2 and 20-21: in L-conformation Nucleotides 3-5 and 16-17: G-stretch Nucleotides 8-9, 12-13 and 15-16: CG-motif
<400> 23
   gggggaacga tcgtcggaag gt 22
<210> 24
   <211> 21
   <212> DNA
   <213> Artificial Sequence
<220>
   <223> synthetic oligodeoxynucleotide, CKm460
<220>
   <221> misc_feature
   <223> Nucleotides 1-2 and 19-20: in L-conformation Nucleotides 4-5, 15-16 and 18: G-stretch Nucleotides 7-8, 11-12 and 14-15: CG-motif
<400> 24
   ggaggacgat cgtcggaggg t 21
<210> 25
   <211> 22
   <212> DNA
   <213> Artificial Sequence
<220>
   <223> synthetic oligodeoxynucleotide, CKm475
<220>
   <221> misc_feature
   <223> Nucleotides 1-7 and 17-21: in L-conformation Nucleotides 15-16: G-stretch Nucleotides 7-8, 11-12 and 14-15: CG-motif
<400> 25
   cccccccgat cgtcgggggg gt 22
<210> 26
   <211> 22
   <212> DNA
   <213> Artificial Sequence
<220>
   <223> synthetic oligodeoxynucleotide, CKm477
<220>
   <221> misc_feature
   <223> Nucleotides 1-2 and 20-21: in L-conformation Nucleotides 3-5 and 16-19: G-stretch
<400> 26
   gggggagcat gctgcggggg gt 22
<210> 27
   <211> 21
   <212> DNA
   <213> Artificial Sequence
<220>
   <223> synthetic oligodeoxynucleotide, CKm463
<220>
   <221> misc_feature
   <223> Nucleotides 1-2 and 19-20: in L-conformation Nucleotides 5 and 15-16: G-stretch Nucleotides 7-8, 11-12 and 14-15: CG-motif
<400> 27
   ggaagacgat cgtcggaagg t 21
<210> 28
   <211> 21
   <212> DNA
   <213> Artificial Sequence
<220>
   <223> synthetic oligodeoxynucleotide, CKm504
<220>
   <221> misc_feature
   <223> Nucleotides 1-2 and 19-20: in L-conformation Nucleotides 3-5 and 15-18: G-stretch Nucleotides 11-12 and 14-15: CG-motif
<400> 28
   gggggagcat cgtcgggggg t 21
<210> 29
   <211> 20
   <212> DNA
   <213> Artificial Sequence
<220>
   <223> synthetic oligodeoxynucleotide, CKm510
<220>
   <221> misc_feature
   <223> Nucleotides 1-2 and 18-19: in L-conformation Nucleotides 3-5, 14-15 and 17: G-stretch Nucleotides 6-7, 10-11 and 13-14: CG-motif
<400> 29
   gggggcgatc gtcggagggt 20
<210> 30
   <211> 24
   <212> DNA
   <213> Artificial Sequence
<220>
   <223> synthetic oligodeoxynucleotide, CKm509
<220>
   <221> misc_feature
   <223> Nucleotides 1-6 and 19-23: in L-conformation Nucleotides 3-5 and 17-18: G-stretch Nucleotides 9-10, 13-14 and 16-17: CG-motif
<400> 30
   ccccccctcg atcgtcgggg gggt 24
<210> 31
   <211> 34
   <212> DNA
   <213> Artificial Sequence
<220>
   <223> synthetic oligodeoxynucleotide, CKm532
<220>
   <221> misc_feature
   <223> Nucleotides 32-33: in L-conformation Nucleotides 1-4 and 21-24: G-stretch Nucleotides 13-14, 20-21 and 25-26: CG-motif
<400> 31
   ggggtcatta aacgttcttc ggggcgttct tttt 34
<210> 32
   <211> 35
   <212> DNA
   <213> Artificial Sequence
<220>
   <223> synthetic oligodeoxynucleotide, CKm527
<220>
   <221> misc_feature
   <223> Nucleotides 33-34: in L-conformation Nucleotides 1-4, 21-24 and 26-30: G-stretch Nucleotides 13-14, 20-21 and 25-26: CG-motif
<400> 32
   ggggtcatta aacgttcttc ggggcggggg ttttt 35
<210> 33
   <211> 33
   <212> DNA
   <213> Artificial Sequence
<220>
   <223> synthetic oligodeoxynucleotide, CKm501
<220>
   <221> misc_feature
   <223> Nucleotides 31-32: in L-conformation Nucleotides 1-3 and 20-23: G-stretch Nucleotides 12-13, 19-20 and 24-25: CG-motif
<400> 33
   gggtcattaa acgttcttcg gggcgttctt ttt 33
<210> 34
   <211> 34
   <212> DNA
   <213> Artificial Sequence
<220>
   <223> synthetic oligodeoxynucleotide, CKm534
<220>
   <221> misc_feature
   <223> Nucleotides 32-33: in L-conformation Nucleotides 1-3 and 21-24: G-stretch Nucleotides 13-14, 20-21 and 25-26: CG-motif
<400> 34
   gggtcattaa aacgttcttc ggggcgttct tttt 34
<210> 35
   <211> 37
   <212> DNA
   <213> Artificial Sequence
<220>
   <223> synthetic oligodeoxynucleotide, CKm520
<220>
   <221> misc_feature
   <223> Nucleotides 35-36: in L-conformation Nucleotides 1-4 and 28-32: G-stretch Nucleotides 13-14, 20-21 and 27-28: CG-motif
<400> 35
   ggggtcatta aacgttcttc gttcttcggg ggttttt 37
<210> 36
   <211> 31
   <212> DNA
   <213> Artificial Sequence
<220>
   <223> synthetic oligodeoxynucleotide, CKm535
<220>
   <221> misc_feature
   <223> Nucleotides 29-30: in L-conformation Nucleotides 17-20 and 22-26: G-stretch Nucleotides 9-10, 16-17 and 21-22: CG-motif
<400> 36
   tcattaaacg ttcttcgggg cgggggtttt t 31
<210> 37
   <211> 33
   <212> DNA
   <213> Artificial Sequence
<220>
   <223> synthetic oligodeoxynucleotide, CKm528
<220>
   <221> misc_feature
   <223> Nucleotides 31-32: in L-conformation Nucleotides 1-3 and 20-23: G-stretch Nucleotides 13-14, 19-20 and 24-25: CG-motif
<400> 37
   gggtcattaa aacgttctcg gggcgttctt ttt 33
<210> 38
   <211> 30
   <212> DNA
   <213> Artificial Sequence
<220>
   <223> synthetic oligodeoxynucleotide, CKm339
<220>
   <221> misc_feature
   <223> Nucleotides 6-7 and 20-23: G-stretch Nucleotides 12-13, 19-20 and 24-25: CG-motif Nucleotides 1-2 and 29-30: phosphothioate bonds
<400> 38
   tcattggaaa acgttcttcg gggcgttctt 30
<210> 39
   <211> 36
   <212> DNA
   <213> Artificial Sequence
<220>
   <223> synthetic oligodeoxynucleotide, CKm498
<220>
   <221> misc_feature
   <223> Nucleotides 34-35: in L-conformation Nucleotides 6-7 and 27-33: G-stretch Nucleotides 12-13, 19-20 and 26-27: CG-motif
<400> 39
   tcattggaaa acgttcttcg ttcttcgggg gggttt 36
<210> 40
   <211> 28
   <212> DNA
   <213> Artificial Sequence
<220>
   <223> synthetic oligodeoxynucleotide, CKm536
<220>
   <221> misc_feature
   <223> Nucleotides 26-27: in L-conformation Nucleotides 1-3 and 16-19: G-stretch Nucleotides 8-9, 15-16 and 20-21: CG-motif
<400> 40
   gggaaaacgt tcttcggggc gttctttt 28
<210> 41
   <211> 33
   <212> DNA
   <213> Artificial Sequence
<220>
   <223> synthetic oligodeoxynucleotide, CKm499
<220>
   <221> misc_feature
   <223> Nucleotides 31-32: in L-conformation Nucleotides 6-8 and 20-23: G-stretch Nucleotides 12-13, 19-20 and 24-25: CG-motif
<400> 41
   tcattgggaa acgttcttcg gggcgttctt ttt 33
<210> 42
   <211> 33
   <212> DNA
   <213> Artificial Sequence
<220>
   <223> synthetic oligodeoxynucleotide, CKm533
<220>
   <221> misc_feature
   <223> Nucleotides 31-32: in L-conformation Nucleotides 1-3, 15-17 and 20-23: G-stretch Nucleotides 12-13, 19-20 and 24-25: CG-motif
<400> 42
   gggtcattaa acgtgggtcg gggcgttctt ttt 33
<210> 43
   <211> 33
   <212> DNA
   <213> Artificial Sequence
<220>
   <223> synthetic oligodeoxynucleotide, CKm500
<220>
   <221> misc_feature
   <223> Nucleotides 31-32: in L-conformation Nucleotides 9-11 and 20-23: G-stretch Nucleotides 12-13, 19-20 and 24-25: CG-motif
<400> 43
   tcattaaagg gcgttcttcg gggcgttctt ttt 33
<210> 44
   <211> 25
   <212> DNA
   <213> Artificial Sequence
<220>
   <223> synthetic oligodeoxynucleotide, CKm521
<220>
   <221> misc_feature
   <223> Nucleotides 23-24: in L-conformation Nucleotides 1-3 and 14-17: G-stretch Nucleotides 6-7, 13-14 and 18-19: CG-motif
<400> 44
   gggaacgttc ttcggggcgt ctttt 25
<210> 45
   <211> 23
   <212> DNA
   <213> Artificial Sequence
<220>
   <223> synthetic oligodeoxynucleotide, CKm502
<220>
   <221> misc_feature
   <223> Nucleotides 21-22: in L-conformation Nucleotides 1-3 and 12-15: G-stretch Nucleotides 4-5, 11-12 and 16-17: CG-motif
<400> 45
   gggcgttctt cggggcgtct ttt 23
<210> 46
   <211> 31
   <212> DNA
   <213> Artificial Sequence
<220>
   <223> synthetic oligodeoxynucleotide, CKm374
<220>
   <221> misc_feature
   <223> Nucleotides 1-2 and 29-30: in L-conformation Nucleotides 6-7 and 20-23: G-stretch Nucleotides 12-13, 19-20 and 24-25: CG-motif
<400> 46
   tcattggaaa acgttcttcg gggcgttctt t 31
<210> 47
   <211> 33
   <212> DNA
   <213> Artificial Sequence
<220>
   <223> synthetic oligodeoxynucleotide, CKm497
<220>
   <221> misc_feature
   <223> Nucleotides 31-32: in L-conformation Nucleotides 6-7 and 27-30: G-stretch Nucleotides 12-13, 19-20 and 26-27: CG-motif
<400> 47
   tcattggaaa acgttcttcg ttcttcgggg ttt 33
<210> 48
   <211> 33
   <212> DNA
   <213> Artificial Sequence
<220>
   <223> synthetic oligodeoxynucleotide, CKm524
<220>
   <221> misc_feature
   <223> Nucleotides 31-32: in L-conformation Nucleotides 1-3 and 21-24: G-stretch
<400> 48
   gggtcattaa agcttcttgc ggggcttctt ttt 33
<210> 49
   <211> 31
   <212> DNA
   <213> Artificial Sequence
<220>
   <223> synthetic oligodeoxynucleotide, CKm525
<220>
   <221> misc_feature
   <223> Nucleotides 1-2 and 29-30: in L-conformation Nucleotides 6-7 and 21-24: G-stretch
<400> 49
   tcattggaaa agcttcttgc ggggcttctt t 31
<210> 50
   <211> 27
   <212> DNA
   <213> Artificial Sequence
<220>
   <223> synthetic oligodeoxynucleotide, CKm526
<220>
   <221> misc_feature
   <223> Nucleotides 25-26: in L-conformation Nucleotides 1-3 and 15-18: G-stretch Nucleotides 7-8, 14-15 and 19-20: CG-motif
<400> 50
   gggaaacgtt cttcggggcg ttctttt 27
<210> 51
   <211> 35
   <212> DNA
   <213> Artificial Sequence
<220>
   <223> synthetic oligodeoxynucleotide, CKm537
<220>
   <221> misc_feature
   <223> Nucleotides 33-34: in L-conformation Nucleotides 1-4, 16-18, 21-24 and 26-30: G-stretch Nucleotides 13-14, 20-21 and 25-26: CG-motif
<400> 51
   ggggtcatta aacgtgggtc ggggcggggg ttttt 35
<210> 52
   <211> 32
   <212> DNA
   <213> Artificial Sequence
<220>
   <223> synthetic oligodeoxynucleotide, CKm538
<220>
   <221> misc_feature
   <223> Nucleotides 30-31: in L-conformation Nucleotides 1-4, 9-12, 16-19 and 23-27: G-stretch Nucleotides 8-9, 15-16 and 22-23: CG-motif
<400> 52
   ggggaaacgg ggttcggggt tcgggggttt tt 32

## Description

### FIELD OF THE INVENTION

The present invention relates to a nucleic acid molecule and its use for the modulation of the immune system.

### BACKGROUND OF THE INVENTION

An emerging strategy to fight complex diseases, such as cancer, infectious diseases, allergy and asthma, is to utilize the patient's immune system. It is known that the immune system or its activity can be modulated by specific DNA sequences. Most known immunomodifying short DNA sequences contain an unmethylated cytosine guanine motif (CG motif) which has been described by Krieg et al. (Nature 1995 374: 6522 546-549). The occurrence of unmethylated CG motifs is substantially suppressed in the genome of eukaryotes compared to prokaryotes or viruses. Therefore, DNA molecules containing such a motif have evolved as a natural "danger signal" and trigger the immune system in the fight against prokaryotic or viral pathogens. This can be exploited therapeutically or prophylactically to treat or prevent infectious as well as non-infectious diseases.

DNA constructs comprising unmethylated CG motifs are able to elicit a considerable physiological effect by strongly stimulating effector cells of the innate immune system including dendritic cells, macrophages, natural killer (NK) and NKT cells. Unmethylated CG motifs are detected by the innate immune pattern recognition receptor Toll-like receptor (TLR) 9. While the exact recognition mechanism is not yet fully understood, significant progress in unraveling the underlying pathways has been made (A. Krieg, Nat. Rev. Drug Disc., 5:471-484, 2006). It is assumed that upon binding of DNA constructs containing unmethylated CGs to the receptor, multiple signal cascades are activated in responding cells. By upregulation of characteristic surface molecules and secretion of cytokines, adaptive immunity with a predominant Th1 pattern is induced. Such constructs can be used in combination with, for example, antibodies, chemotherapy or radiation therapy, vaccines or cytokines. Allergic diseases and asthma are mostly Th2-mediated. By increasing the ratio of Th1/Th2, the Th2-mediated responses are attenuated and thereby these types of diseases can be treated or prevented.

Surface molecules include, for example, CD40, CD69, CD80 or CD86, depending on the specific cell type analyzed. Secretion of cytokines is also characteristic for distinct cell types; cytokines include, for example, macrophage inflammatory proteins (MIP)-1alpha, MIP-1beta, interleukin (IL)-6, IL-8, interferon (IFN)-alpha, tumor necrosis factor (TNF)-alpha, IFN-gamma, monocyte chemotactic protein (MCP)-1 or IFN-gamma-induced protein of 10 kDa (IP-10).

In order to prevent or treat diseases, vaccination has been proven as a very effective approach. To ensure a strong and durable immune response, adjuvants capable of stimulating antigen-presenting cells such as dendritic cells, are usually administered together with the antigen, and for that purpose TLR9 agonists have been shown to be potent immunostimulants.

Independently of any explanations of the underlying mechanisms by which unmethylated CG motifs influence or modulate an immune response, many approaches were developed for modulation of the immune system by using such motifs. The WO 1998/018810 discloses that immunostimulatory sequences containing unmethylated CG motifs are even more effective when they are part of a single strand. However, administering an open-chained single-stranded DNA molecule is not practicable due to the quick degradation of single-stranded nucleic acids. Consequently, different methods for the protection of single- or double-stranded DNA constructs comprising an unmethylated CG motif were developed.

To achieve resistance against the degradation by DNA nucleases the phosphodiester bonds in the backbone of a nucleic acid polymer are frequently modified to phosphorothioates. Besides a somewhat less stimulatory activity of such phosphorothioate-protected nucleic acids clinical trials within the last years showed that the toxicity of a phosphorothioate-protection exclude or severely limit such nucleic acids from any use in pharmaceutical compositions or medicaments.

Another approach to protect DNA sequences comprising a CG motif is disclosed for example in EP 1 196 178. This document discloses short deoxyribonucleic acid molecules, comprising a partially single-stranded, dumbbell-shaped, covalently closed sequence of nucleotide residues comprising CG motifs ("dSLIM"). According to the disclosure of the EP 1 196 178 the CG motifs are located within the single-stranded loops at both ends of the double-stranded stem of the disclosed molecule or within the double-stranded stem. The single-stranded hairpin loops protect a double-stranded stem from degradation by DNA nucleases within or outside of the cell.

Document WO 2010/039137 discloses immune regulatory oligonucleotides as antagonists for TLR mediated diseases having one or more chemical modifications in the sequence flanking an immune stimulatory motif and/or in an oligonucleotide motif that would be immune stimulatory but for the modification. Thus, the intention of the disclosed oligonucleotides of WO 2010/039137 is to suppress an immune response caused by TLRs.

WO 2005/042018 describes new so-called C-class CpG oligonucleotides, wherein a c-class oligonucleotide is characterised by CpG sequences, generally positioned at or near the 5' end or 3' end of the molecule, and a GC-rich palindrome motif, generally positioned at or near the other end of the molecule. The document discloses variations of the palindromic sequence of a c-class DNA.

Document DE 102 11 558 A1 discloses an RNA molecule comprising at least one L-nucleotide. The RNA molecules are used for the activation of specific signal cascades, respectively the activation of transcription factors.

In US 2008/260820 protease-resistant polypeptides are disclosed, which can be administered in combination with L-nucleosides for the treatment of viral infections. The L-nucleosides (which are nucleoside analogues) are simply used as additives in combination with the polypeptide. They are not incorporated into a DNA construct. The teaching of US 2008/260820 only discloses a method of treatment of a disease comprising administration of a modified polypeptide.

Document EP 2 333 091 A2 discloses DNA vectors/fragments containing one or more immunostimulatory RNA elements. The plasmids eliminate all extraneous sequences, incorporate a novel antibiotic free short RNA based selectable marker, increase eukaryotic expression using a novel chimeric promoter, improve yield and stability during bacterial production, and improve immunostimulation. These vectors are utilised in immunisation to elicit improved immune responses or therapy to induce type 1 interferon production.

In US 2002/095033 A1 L-nucleosides and methods of their manufacture are disclosed. Although these L-nucleosides can be used to treat infections or cancer, for example, this document does not deal with CG-containing DNA constructs.

Document EP 2 246 433 A1 discloses a concatemeric DNA molecule comprising CG sequences used for immunomodulation. This document discloses a structure comprising several subunits, which are covalently bound to each other.

### BRIEF SUMMARY OF THE DISCLOSURE

With regard to the state of the art it is an objective of the present disclosure to provide alternative immunomodulating DNA constructs being stable after transfer into eukaryotic cells and avoiding harmful side effects.

The present invention teaches a DNA construct according to claim 1.

As a further embodiment the construct comprises inter- and/or intramolecular base-pairs and at least one unpaired, single-stranded region.

Furthermore, a multimeric construct is provided, wherein at least two constructs comprising inter- and/or intramolecular base-pairs and at least one unpaired, single-stranded region ligate to one another.

In addition, the construct may comprise at least one nucleotide in L- or D-conformation which is modified with a functional group selected from the group comprising carboxyl, amine, amide, aldimine, ketal, acetal, ester, ether, disulfide, thiol and aldehyde groups.

The modified nucleotide may be linked to a compound selected from the group comprising peptides, proteins, carbohydrates, antibodies, synthetic molecules, polymers, micro projectiles, metal particles, nanoparticles, micelles, lipid carriers, or a solid phase.

The constructs according to the present disclosure can be used for the treatment of cancer or autoimmune diseases or for the modulation of the immune system.

As a further embodiment of the present disclosure a pharmaceutical composition is provided comprising a DNA construct as described above. The pharmaceutical composition may also comprise a chemotherapeutic.

Furthermore, a vaccine is provided which comprises a DNA construct as described above. Therein, the DNA construct may be comprised as adjuvant.

### DETAILED DESCRIPTION OF THE DISCLOSURE

Within the meaning of the present disclosure a linear open-chained DNA sequence is designated as DNA construct. Said DNA sequence can be single-stranded or partially or completely double-stranded. The term DNA construct does not indicate a limitation of the length of the corresponding DNA sequence. The monomeric units of DNA constructs are nucleotides.

A DNA construct can be manufactured synthetically or be partially or completely of biological origin, wherein a biological origin includes genetically based methods of manufacture of DNA sequences.

L-DNA or nucleotides in L-conformation refer to nucleotides, which comprise L-deoxyribose as the sugar residue instead of the naturally occurring D-deoxyribose. L-deoxyribose is the enantiomer (mirror-image) of D-deoxyribose. DNA constructs partially or completely consisting of nucleotides in L-conformation can be partially or completely single-or double-stranded; however, nucleotides in L-conformation cannot hybridize to nucleotides in D-conformation (Hauser et al., Nucleic Acid Res. 2006 34: 5101-11). L-DNA is equally soluble and selective as D-DNA. Yet, L-DNA is resistant towards degradation by naturally occurring enzymes, especially exonucleases, so L-DNA is protected against biological degradation (Urata et al., Nucleic Acids Res. 1992 20: 3325-32). Therefore, L-DNA is very widely applicable.

A "stem" according to the present disclosure shall be understood as a DNA double strand formed by base pairing either within the same DNA molecule (which is then partially self-complementary) or within different DNA molecules (which are partially or completely complementary). Intramolecular base-pairing designates base-pairing within the same molecules and base-pairing between different DNA molecules is termed as intermolecular base-pairing.

A "loop" within the meaning of the present disclosure shall be understood as an unpaired, single-stranded region either within or at the end of a stem structure. A "hairpin" is a distinct combination of a stem and a loop, which occurs when two self-complementary regions of the same DNA molecule hybridize to form a stem with an unpaired loop. A dumbbell-shape describes a linear DNA construct with hairpins at both ends flanking a stem region. Thus, a "linear DNA construct" within the context of the present disclosure describes either a linear open-chained DNA construct comprising single or double-stranded DNA or a linear dumbbell-shaped DNA construct comprising single stranded loops at both ends of a double stranded DNA stem.

The term "DNA end", whether meaning a 5'- or 3'end of a DNA single strand, refers not only to the terminal nucleotide, but comprises the terminal five nucleotides or even the last threes nucleotides with regard to the respective DNA end. A modification of a DNA end relates to at least one of the respective nucleotides.

A "G stretch" shall be understood within the meaning of the present disclosure as a sequence of at least three consecutive deoxyguanosines.

A "solid phase" to which the nucleotides are covalently or non-covalently attached refers to, but is not restricted to, a column, a matrix, beads, glass including modified or functionalized glass, silica or silica-based materials including silicon and modified silicon, plastics (comprising polypropylene, polyethylene, polystyrene and copolymers of styrene and other materials, acrylics, polybutylene, polyurethanes etc.), nylon or nitrocellulose, resins, polysaccharides, carbon as well as inorganic glasses, metals, nanoparticles, and plastics. Thus, microtiter plates are also within the scope of a solid phase according to the present disclosure.

Immunomodulation according to the present disclosure refers to immunostimulation and immunosuppression. Immunostimulation means preferentially that effector cells of the immune system are stimulated in order to proliferate, migrate, differentiate or become active in any other form. B cell proliferation for instance can be induced without co-stimulatory signals by immunostimulatory DNA molecules, which normally require a co-stimulatory signal from helper T-cells.

Immunosuppression on the other hand shall be understood as reducing the activation or efficacy of the immune system. Immunosuppression is generally deliberately induced to prevent for instance the rejection of a transplanted organ, to treat graft-versus-host disease after a bone marrow transplant, or for the treatment of autoimmune diseases such as, for example, rheumatoid arthritis or Crohn's disease.

In this context, immunomodulation may also refer to the influence of the nature or the character of an immune reaction, either by affecting an immune reaction which is still developing or maturing or by modulating the character of an established immune reaction.

The term "vaccination" used in this disclosure refers to the administration of antigenic material (a vaccine) to produce immunity to a disease. Vaccines can prevent or ameliorate the effects of infection by many pathogens such as viruses, fungi, protozoan parasites, bacteria but also of allergic diseases and asthma, as well as of tumors. Vaccines typically contain one or more adjuvants, e.g. immunostimulatory nucleic acids, used to boost the immune response. Vaccination is generally considered to be the most effective and cost-effective method of preventing infectious and other diseases.

The material administered can, for example, be live but weakened forms of pathogens (bacteria or viruses), killed or inactivated forms of these pathogens, purified material such as proteins, nucleic acids encoding antigens, or cells such as tumor cells or dendritic cells. In particular, DNA vaccination has recently been developed. DNA vaccination works by insertion (and expression, triggering immune system recognition) of DNA encoding antigens into human or animal cells. Some cells of the immune system that recognize the proteins expressed will mount an attack against these proteins and against cells expressing them. One advantage of DNA vaccines is that they are very easy to produce and store. In addition, DNA vaccines have a number of advantages over conventional vaccines, including the ability to induce a wider range of immune response types.

Vaccination can be used as a prophylactic approach, leading to immunity against the antigen in the vaccinated, healthy individual upon exposure to the antigen. Alternatively, a therapeutic vaccination can cause an improved response of the immune system of the vaccinated, diseased individual, by guiding the immune system of the individual towards the antigens. Both prophylactic and therapeutic vaccination can be applied to humans as well as animals.

The term "gene therapy" used in this disclosure refers to the transient or permanent genetic modification (e.g. insertion, alteration, or removal of genes) of an individual's cells and/or biological tissues in order to treat diseases, such as tumors or autoimmune diseases. The most common form of gene therapy involves the insertion of functional genes into an unspecified genomic location in order to replace a mutated gene, but other forms involve directly correcting the mutation or modifying a normal gene that enables a viral infection or even transferring a gene or a gene fragment into a cell for its transcription.

"Autologous gene therapy" refers to using tissues or cells of the selfsame individual. The isolated cells or tissues will be modified by gene therapy and reintroduced into the donor. In contrast, "allogenic gene therapy" refers to using cells for gene therapy from an individual other than the acceptor individual. After genetic modification, the allogenic cells are introduced into the acceptor.

The term *"ex-vivo* gene therapy" refers to a therapy approach in which cells from an individual, e.g. hematopoietic stem cells or hematopoietic progenitor cells, are genetically modified *ex vivo* and subsequently introduced to the individual to be treated. The term *"in-vivo* gene therapy" refers to a therapy approach in which cells from an individual, e.g. hematopoietic stem cells or hematopoietic progenitor cells, are genetically modified *in vivo*, using viral vectors or other expression constructs for example.

Gene therapy may also be classified into "germ line gene therapy" and "somatic gene therapy". In case of "germ line gene therapy", germ cells, i.e., sperm or eggs, are genetically modified. The genetic changes are ordinarily integrated into their genomes. Therefore, the change due to therapy would be heritable and would be passed on to later generations. This approach is useful for treatment of genetic disorders and hereditary diseases. In case of "somatic gene therapy", the therapeutic genes are transferred into the somatic cells of an individual. Any modifications and effects will be restricted to the individual only, and will not be inherited by the individual's offspring or later generations

The term "cancer" comprises cancerous diseases or a tumor being treated or prevented that is selected from the group comprising, but not limited to, mammary carcinomas, melanoma, skin neoplasms, lymphoma, leukemia, gastrointestinal tumors, including colon carcinomas, stomach carcinomas, pancreas carcinomas, colon cancer, small intestine cancer, ovarial carcinomas, cervical carcinomas, lung cancer, prostate cancer, kidney cell carcinomas and/or liver metastases.

Autoimmune diseases according to the present disclosure comprise rheumatoid arthritis, Crohn's disease, systemic lupus (SLE), autoimmune thyroiditis, Hashimoto's thyroiditis, multiple sclerosis, Graves' disease, myasthenia gravis, celiac disease and Addison's disease.

The present disclosure provides a linear open-chained DNA sequence comprising at least one CG motif and at least one nucleotide in L-conformation. Due to the partial/L-conformation the DNA cannot act as substrates to naturally occurring, D-conformation-specific DNA-degrading enzymes. Thereby, the DNA constructs of the present invention are protected against enzymatic degradation without having to use a phosphorothioate backbone which has been shown to be toxic. In addition, the DNA constructs only consist of a minimum number of nucleotides which makes them small and thereby siginificantly improves their uptake by the patient's cells.

The effect of CG-containing DNA constructs depends on their interaction with TLR9, and DNA-protein interaction depends on the conformation of both DNA and protein. Since the chirality of the single molecules is decisive for the conformation of the resulting polymer it was not known whether a DNA molecule in partial or complete L-conformation would be capable of binding to and activating TLR9. Experimental data demonstrate that such protected DNA molecules are surprisingly suitable for the induction of an immune response. As shown in the examples and figures, at least a partial change in chirality of single nucleotides obviously still allows binding to and activation of TLR9. Therefore, DNA molecules with CG motifs and nucleotides in L-conformation can be used for immunomodulation.

Surprisingly, the induced stimulation pattern differs from the stimulation pattern induced by the molecule disclosed in EP 1 196 178 disclosing the dumbbell shaped molecule comprising CG motifs in the single-stranded loops at both ends of the molecule or in the double-stranded stem ("dSLIM"), as can be seen in the figures, even when employing identical nucleotide sequences.

The DNA construct can be single-stranded or partially or completely double-stranded. This includes base-pairing within the same molecule (intramolecular) or within different molecules (intermolecular) or any combination thereof. It is also possible that the construct comprises at least one unpaired, single-stranded region. As a further embodiment, hairpin structures are included. Due to the partial or complete L-conformation, a longer half life of the construct is ensured as nucleotides in L-conformation are not subject to degradation.

It is also intended that at least two molecules, which are single-stranded or partially or completely double-stranded can ligate to one another to form multimeric constructs. These multimeric constructs thus incorporate at least as many CG motifs as ligation partners, tightly packed within one molecule, and are therefore expected to elicit a considerable immune response. The resulting single-stranded or partially or completely double-stranded multimeric constructs can either be covalently closed comprising nucleotides in L-conformation within the molecule or open multimeric constructs comprising nucleotides in L-conformation at or near the 5'- and/or the 3'-end for protection against enzymatic degradation.

According to the present disclosure the CG motif/s is/are located within the single-stranded and/or double-stranded region of the construct. As has been disclosed in EP 1 196 178, CG motifs are capable of eliciting an immune response whether they are included within the single-stranded or within the double-stranded region of the molecule.

The disclosure further comprises chemical modifications of at least one nucleotide in L- or D-conformation with a functional group selected from the group comprising carboxyl, amine, amide, aldimine, ketal, acetal, ester, ether, disulfide, thiol and aldehyde groups. This allows coupling of the DNA construct to a compound selected from the group comprising peptides, proteins, lipids, vesicles, micelles, carbohydrates, antibodies, synthetic molecules, polymers, micro projectiles, metal particles, nanoparticles or a solid phase by, for example, adsorption, covalent or ionic bonding. The modification can be specifically selected for the respective purpose. The construct can thereby be used, for example, to shuttle other molecules to the specific cell responding to the CG motif/s incorporated. In addition, it is possible by such modifications to couple the construct to micro projectiles which can be used to transfer the construct into the cell. The construct can also be coupled to a solid phase, e. g. a microtiter plate.

Th1-biased activation involves the activation of NK cells and cytotoxic T cells and these immune responses can be exploited for cancer therapy. Since DNA constructs containing unmethylated CG motifs preferably lead to Th1 activation, the constructs of the present disclosure can be used for treating cancer. Numerous clinical trials are ongoing involving TLR9 agonists for treatment of cancer. Such molecules have been effectively administered alone or in combination with, for example, radiation therapy, surgery, chemotherapy and cryotherapy (Krieg, J. Clin. Invest.2007 117: 1184-94). Due to their potent immunomodulation, their small size and their stability the constructs of the present disclosure are expected to be highly advantageous in this regard. In addition, their distinct immunological profile distinguishes them from other, less advantageous TLR9 ligands, and this profile can be exploited for cancer-specific treatment.

On the other hand, TLR9 agonists are also involved in the generation of regulatory T cells and can thus be used for the treatment of autoimmune diseases. The route of administration seems to be one variable determining the effect of DNA constructs containing CG motifs *in vivo* (Krieg, J. Clin. Invest.2007 117: 1184-94).

The immunostimulatory effect of such DNA molecules containing CG-motifs has been shown to improve the efficacy of standard therapeutical approaches such as chemotherapeutics, in cancer therapy. Therefore, pharmaceutical compositions, which comprise the constructs of the present disclosure, are also provided. Again, the advantageous features of the constructs of the present disclosure compared with the TLR9 agonists of the state of the art makes the constructs of the present disclosure promising tools for treatment of diseases such as cancer, infectious diseases, allergies and asthma. The treatment of allergies and asthma (mostly Th2-mediated) thereby benefits from the preference of Th1 activation.

Since TLR9 agonists have been shown to be potent adjuvants in vaccines, vaccines comprising the DNA constructs of the present disclosure are also provided. The constructs of the present disclosure only comprise the relevant sequences for TLR9 stimulation and are stable due to the L-nucleotide modification. Therefore, side effects due to non-relevant sequences can be avoided. The longer half-life of the molecule ensures efficient stimulation so that a strong immune response is expected.

The DNA molecules of the present disclosure were produced by using a synthesis column and the respective nucleotides (Beta-L-deoxy "NT" (n-bz) CED phosphoramidite; "NT" stands for adenosine, cytidine, guanosine or thymidine). The DNA molecules were subsequently purified by HPLC.

To reveal the effect of using DNA with L-ribose instead of D-ribose, the following DNA molecules were used for initial experiments described herein (Table 1).

**Table 1: Sequences of the non-coding immunostimulatory DNA constructs and the controls.**

| **SEQ ID NO** | **Name** | **Sequence (5'-3')** | **Modified nucleotides** |
|---|---|---|---|
| 1 | lin 30L2 | TCATTGGAAAACGTTCTTC GGGGCGTTCTT | none |
| 2 | CKm336, Lin L | TGGAAAACGTTCTTCGGGG CGTTCTTT | completely in L-conformation, except for the last T |
| 3 | CKm337, CKm374 | TCATTGGAAAACGTTCTTC GGGGCGTTCTTT | 1, 2, 29 and 30 in L-conformation |
| 4 | CKm338, linPT | TCATTGGAAAACGTTCTTC GGGGCGTTCTTT | all but the last phosphodiester bonds modified to phosphorothioates |
| 5 | CKm339, Lin2tPT | TCATTGGAAAACGTTCTTC GGGGCGTTCTTT | first two as well as second- and third-to-last phosphordiester bonds modified to phosphorothioates |

Experiments using the sequences of Table 1 showed that L-ribose protected linear sequences containing CG motifs are able to stimulate the immune system and the induced immune response differs clearly from the immune response induced by dSLIM as disclosed in EP 1 196 178. Thus, a modified sequence called ODN2216 (GGGGGAC-GATCGTCGGGGGG; SEQ ID 6) and modifications thereof were used to investigate the influence of structural difference like the influence of G-stretches with regard to their presence, length and position, the spacing between CG-motifs and the distance between L-ribose nucleotides and CG-motifs respectively G-stretches.

Table 2 summarizes the used sequences and their effect an IFN-alpha and IP-10 secretion in comparison to ODN2216 having the first two and last six nucleotides modified with phosphorothioate, wherein bold letters represent 1-ribose comprising nucleotides, italic letters refer to a G-stretch and underlined letters refer to a CG-motif. A dash shall place the respective sequence in place for comparison with CKm508, but does neither indicate a structural nor a functional modification of the sequence.

**Table 2: Effect of indicated sequences on IFN-alpha and IP-10 secretion in comparison to ODN2216 (ODN2216 having the first two and last six nucleotides modified with phosphorothioate).**

| **Stimulator** | **MW IFN-a (% of ODN2216)** | **MW IP-10 (% of ODN2216** | **Sequenz** | **SEQ ID NO** |
|---|---|---|---|---|
| CKm508 | 122,5093347 | 238,1577436 | GGGGGGGACGATCGTCGGGGG GGGT | 7 |
| CKm458 | 105,7141511 | 204,6403984 | GG--AGGACGATCGTCGGGG-- GGT | 8 |
| CKm481 | 88,91417043 | 189,6025669 | GG--GGGACGATCGTCGGG--- GGT | 9 |
| CKm461 | 86,89157541 | 205,8046048 | GG--AAGACGATCGTCGGGG-- GGT | 10 |
| CKm361-2 | 84,31376637 | 238,7118706 | GG--GGGACGATCGTCGGGG-- GGT | 11 |
| CKm479 | 83,60788319 | 176,7812306 | GG-GGGGACGATCGTCGGGG-- GGT | 12 |
| CKm503 | 64,99582032 | 258,7302716 | AAGGGGGACGATCGTCGGGGG GAAT | 13 |
| CKm507 | 64,93994028 | 224,6315842 | TTGGGGGACGATCGTCGGGGG GTTT | 14 |
| CKm459 | 62,45886305 | 171,4718235 | GG--GGGACGATCGTCGGAG-- GGT | 15 |
| CKm506 | 56,61452004 | 215,2548473 | GG--GGGACGATCGTGCGGGG- GGT | 16 |
| Ckm478 | 25,22927502 | 137,4229666 | GG--GGGGCGATCGTCGGAA-- GGT | 17 |
| CKm480 | 18,07466107 | 150,4467402 | GG--GGGACGATGCTCGGGG-- GGT | 18 |
| CKm462 | 5,362851611 | 38,31712092 | GG--GGGACGATCGTCGGAA-- GGT | 19 |
| CKm505 | 2,196636001 | 82,40020833 | GG--GGGACGATCGTGCGGG-- GGT | 20 |
| CKm476 | 1,963187635 | 26,31602567 | GG-GGGAACGATCGTCGGAA-- GGT | 21 |
| CKm464 | 1,803712909 | 0,517748538 | GG--CCCCCGATCGTCGGGG-- GGT | 22 |
| CKm476 | 0,981593818 | 39,4740385 | GG-GGGAACGATCGTCGGAA-- GGT | 23 |
| CKm460 | 0,730938569 | 19,07664393 | GG--AGGACGATCGTCGGAG-- GGT | 24 |
| CKm475 | 0,318948048 | 1,703784315 | CCCCCCC--GATCGTCGG- GGGGGT | 25 |
| CKm477 | 0,089962277 | 0,160156357 | GG--GGGAGCATGCTGCGGGG- GGT | 26 |
| CKm463 | 0,024204793 | 8,911634665 | GG--AAGACGATCGTCGGAA-- GGT | 27 |
| CKm504 | 0 | 0,378654915 | GG-GGGAGCATCGTCGGGG- | 28 |
| | | | GGT | |
| CKm510 | 0 | 55,29535465 | GG--GGG-CGATCGTCGGAG-- GGT | 29 |
| CKm509 | 0 | 1,382278733 | CCCCCCCTCGATCGTCGG- GGGGGT | 30 |

Good results were obtained with sequences having at least one G stretch, especially at or near the 3' end. The stimulation is further dependent on the presence of CG-motifs (CKm477), again showing that the stimulation is not an effect of the 1-ribose, but of the CG motifs.

The results obtained with modified sequences of ODN 2216 identifying positive structural components were transferred to the DNA sequence of CKm374. Table 3 shows the results emplying the modified sequences in comparison to dumbbell-shaped dSLIM as disclosed in EP 1 196 178. Again, bold letters represent 1-ribose comprising nucleotides, italic letters refer to a G-stretch, double underlined letters represent phosphorothioate modified nucleotides and underlined letters refer to a GC-motif.

As can be taken from the results in table 3, a G-stretch located directly a the 5' end seems to be advantageous (comp. CKm532 and CKm499). Additionally, using four instead of three deoxyguanosines at the 5' end further increases the stimulation of IFN-alpha and IP-10 (comp. CKm501 and CKm532).

The addition of an additional G-stretch between CG-motifs seems to be beneficial as well (comp. CKm532 and CKm520). The distance between the first and second G-stretch further influences the efficacy of the DNA molecule. Furthermore, employing 1-ribose comprising deoxynucleotides only at or near the 3' seems to yield a sufficient degree of stabilization of the DNA molecule. A good stimulation of IFN-alpha and IP-10 can be observed, which is intended (see below). Because IL-8 has been shown to be responsible for the induction of neo-angiogenesis, it seems to be beneficial, that IL-8 secretion is only induced in small amounts.

Clearly, the presence and carefully chosen position of G stretches in combination with the stabilizing effect of 1-ribose containing deoxynucleotides allows for the production of a DNA molecule which surpasses the stimulation efficiency of the dSLIM molecule.

**Table 3: Comparison of the effect of the indicated sequences on IFN-alpha, IP-10 and IL-8 secretion in comparison to dSLIM.**

| **Stim.** | **Sequence** | **MW IFN-a % dSLIM** | **MW IP-10 % dSLlM** | **MW IL-8 % dSLIM** | **SEQ ID No.** |
|---|---|---|---|---|---|
| CKm532 | *GGGG*TCATTAAACGTTCTTC*GGGG*CGTTCTTTTT | 2300,05 | 297,32 | 261,91 | 31 |
| CKm527 | *GGGG*TCATTAAACGTTCTTC*GGGG*C*GGGGG*TTTTT | 702,07 | 118,20 | 91,58 | 32 |
| CKm501 | *GGG*TCATTAAACGTTCTTC*GGGG*CGTTCTTTTT | 493,25 | 80,23 | 226,82 | 33 |
| CKm534 | *GGG*TCATTAAAACGTTCTTC*GGGG*CGTTCTTTTT | 99,87 | 86,36 | 120,92 | 34 |
| CKm520 | *GGGG*TCATTAAACGTTCTTCGTTCTTC*GGGGG*TTTTT | 52,19 | 24,40 | 378,74 | 35 |
| CKm535 | TCATTAAACGTTCTTC*GGGG*C*GGGGG*TTTTT | 51,32 | 30,49 | 68,75 | 36 |
| CKm528 | *GGG*TCATTAAAACGTTCTC*GGGG*CGTTCTTTTT | 33,71 | 39,01 | 158,76 | 37 |
| CKm339 | TCATTGGAAAACGTTCTTC*GGGG*CGTTCTT | 10,51 | 25,46 | 615,76 | 38 |
| CKm498 | TCATTGGAAAACGTTCTTCGTTCTTC*GGGGGGG*TTT | 8,25 | 23,15 | 170,77 | 39 |
| CKm536 | GGGAAAACGTTCTTCGGGGCGTTCTTTT | 3,13 | 35,64 | 53,03 | 40 |
| CKm499 | TCATTGGGAAACGTTCTTC*GGGG*CGTTCTTTTT | 3,10 | 21,04 | 69,59 | 41 |
| CKm533 | *GGG*TCATTAAACGTGGGTC*GGGG*CGTTCTTTTT | 1,63 | 26,61 | 248,48 | 42 |
| CKm500 | TCATTAAA*GGG*CGTTCTTC*GGGG*CGTTCTTTTT | 1,16 | 9,06 | 70,83 | 43 |
| CKm521 | GGGAACGTTCTTCGGGGCGTCTTTT | 0,15 | 15,09 | 68,07 | 44 |
| CKm502 | GGGCGTTCTTCGGGGCGTCTTTT | 0,13 | 13,57 | 92,81 | 45 |
| CKm374 | TCATTGGAAAACGTTCTTC*GGGG*CGTTCTTT | 0,00 | 11,34 | 77,11 | 46 |
| CKm497 | TCATTGGAAAACGTTCTTCGTTCTTC*GGGG*TTT | 0,00 | 2,57 | 159,64 | 47 |
| CKm524 | GGGTCATTAAAGCTTCTTGC*GGGG*CTTCTTTTT | 0,00 | 9,10 | 37,40 | 48 |
| CKm525 | TCATTGGAAAAGCTTCTTGC*GGGG*CTTCTTT | 0,00 | 2,10 | 27,29 | 49 |
| CKm526 | GGGAAACGTTCTTCGGGGCGTTCTTTT | 0,00 | 13,38 | 79,35 | 50 |
| CKm537 | *GGGG*TCATTAAACGTGGGTC*GGGG*C*GGGGG*TTTTT | 0,00 | 7,45 | 49,24 | 51 |
| CKm538 | *GGGG*AAAC*GGGG*TTC*GGGG*TTC*GGGGG*TTTTT | 0,00 | 2,30 | 32,49 | 52 |

### BRIEF DESCRIPTION OF THE FIGURES

The disclosure will be further illustrated by examples and figures without being limited to the disclosed embodiments. It shows:
- Fig. 1: Agarose gel electrophoresis of DNA constructs after enzymatic digestion
- Fig. 2: GFP intensity after stimulation of a mouse macrophage cell line.
- Fig. 3: MIP-1alpha concentration after stimulating plasmacytoid dendritic cells (PDCs).
- Fig. 4: MIP-1beta concentration after stimulating PDCs.
- Fig. 5: IL-8 concentration after stimulating PDCs.
- Fig. 6: IL-6 concentration after stimulating PDCs.
- Fig. 7: IFN-alpha concentration after stimulating PDCs.
- Fig. 8: TNF-alpha concentration after stimulating PDCs.
- Fig. 9: MCP-1 and IL-8 concentration after stimulating peripheral blood mononuclear cells (PBMCs).
- Fig. 10: Frequency of activated T cells after stimulating PBMCs.
- Fig. 11, 12: IFN-alpha, IP-10 and IL-8 secretion of PBMCs
- Fig. 13: Effect of 1-ribose modified terminal deoxynucleotides on the stimulation of ELAM9 cells
- Fig 14: Immune stimulation of B-cells and PDCs by CKm532 and dSLIM, as compared to the unstimulated state

### DETAILED DESCRIPTION OF THE FIGURES

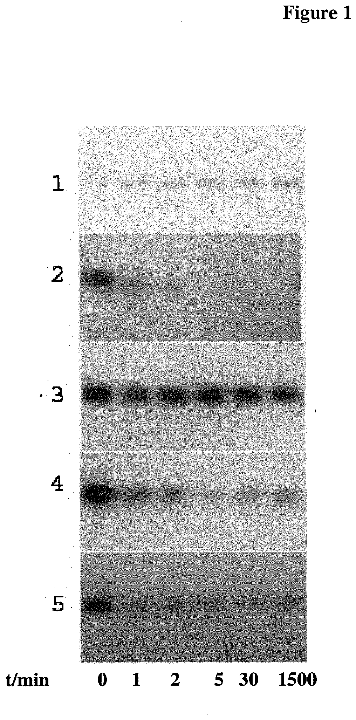

Figure 1 shows a gel of all DNA constructs being subjected to digestion by the T7-Polymerase from the T7 bacteriophage. 6 µg of each DNA construct were incubated with 10 units of T7-Polymerase (total reaction volume: 20 µl). After 0, 1, 2, 5, 30, and 1500 minutes, an aliquot of 3µl of incubation mixture was removed from the sample and diluted with 5µl of formamide-containing Sanger dye. All aliquots were loaded onto a 3% agarose gel, which was run at 100 Volt for 40 minutes.

The unmodified DNA molecule lin-30L2 (lane 2) was found to be completely digested after a 5 minute incubation with T7-Polymerase, while the construct according to the present invention (CKm337; lane 3), as well as dSLIM (lane 1) and the phosphorothioate-modified constructs CKm338 (lane 4) and CKm339 (lane 5) retained significant presence even after 1500 minutes of incubation. In fact, CKm337 showed the highest stability of all molecules tested. Due to its insufficient stability, Lin 30L2 was excluded from further study.

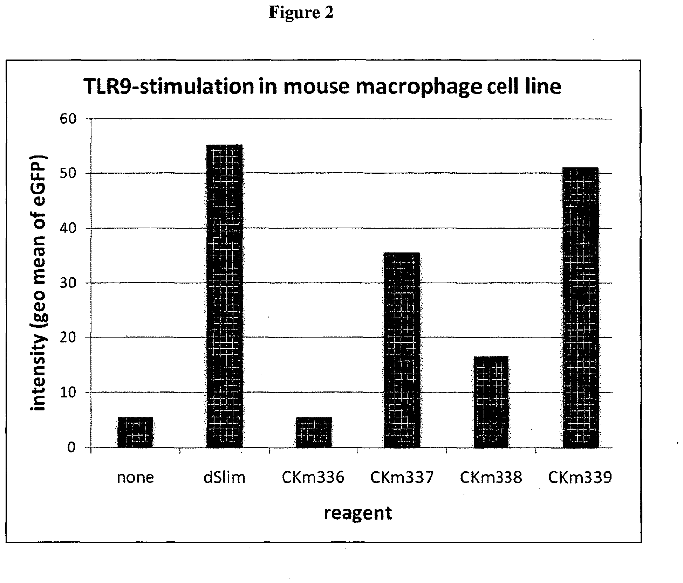

Figure 2 shows the stimulation of ELAM9 cells with different stimulatory DNA constructs. ELAM9 cells are TLR9-positive murine macrophage cells (RAW264) which were stably transfected with d1-eGFP under the control of the human Elastin promoter (hELAM) containing several NPκB response elements. One day after seeding the cells they were stimulated with the depicted DNA constructs (3 µM) for 7 h. The Geo Mean of the GFP intensity was measured by flow cytometry.

The DNA construct with all nucleotides in L-conformation except the last T (CKm336) had no stimulatory capacity. However, the DNA construct with nucleotides in L-conformation at both ends (CKm337) did stimulate GFP-expression. This was rather unexpected since it was not known whether the DNA constructs containing CG motifs with nucleotides in L-conformation would able to bind to and activate TLR9. In addition, CKm337 is expected to be taken up by the cells more easily than dSLIM (molecule disclosed in EP 1 196 178), and to be less toxic than the phosphorothioate-modified constructs (CKm338 and CKm339).

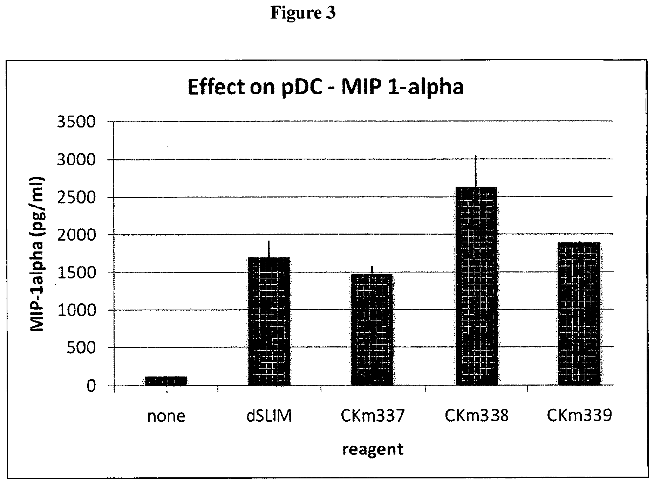

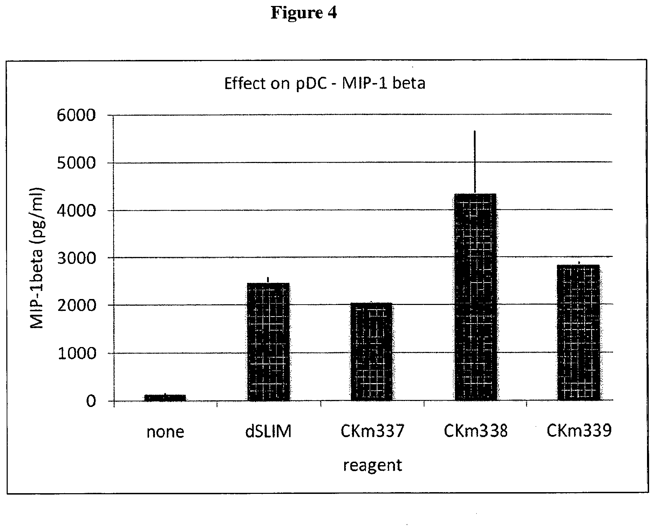

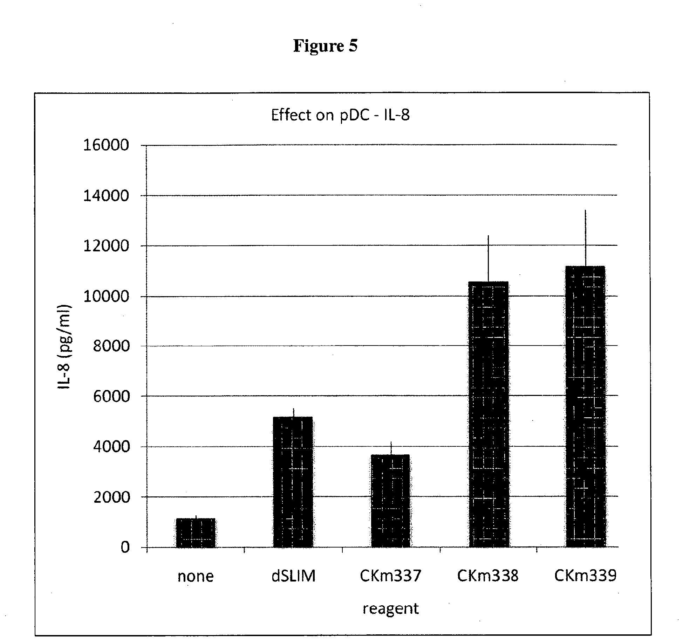

Figure 3 to Figure 8 show the effects of the DNA constructs on pDCs regarding secreted chemokines and cytokines. pDCs were enriched from Ficoll-purified PBMCs using a combined sorting procedure from Miltenyi, Diamond PDC Kit: first, PBMCs were depleted from non-pDCs using the pDC Biotin-Antibody Cocktail from Miltenyi's Kit, then cells were positively sorted for pDCs using the CD304 (BDCA-4) diamond microbeads from the PDC Diamond Kit. PDCs were seeded at 2.5 x 10⁵/ml with 10 ng/ml IL-3 in the medium (RPMI1640, 10% fetal calf serum, 100 Units/ml penicillin, 100 units/ml streptomycine 2 mM glutamine, 37°C, 5% CO₂), and stimulated for 2 days by individual constructs applied at 3 µM.

For determination of the amount of secreted compounds upon cell stimulation, the cleared supernatant of stimulated cells was collected and analysed using a multiplex system (FlowCytomix from eBioscience/Bender MedSystems) or ELISA.

Surprisingly, pDCs stimulated with CKm337 showed a similar effect on MIP-1alpha, -1beta and IL-8 secretion compared to stimulation with dSLIM. MIP-1alpha, -1beta and IL-8 secretion upon stimulation with lin CKm338 and CKm339 was slightly higher (Figure 3, 4 and 5). However, all phosphorothioate-modified constructs inherit several disadvantages as described above.

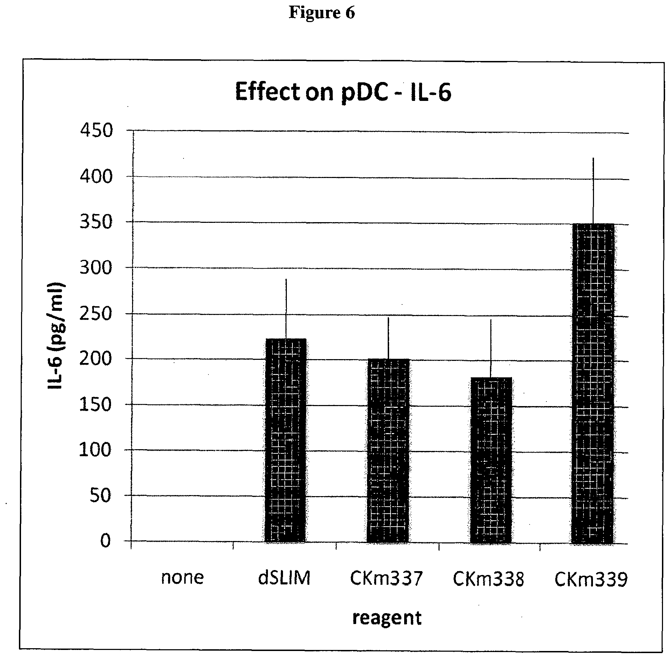

Concerning IL-6 secretion, dSLIM, CKm337 and CKm338 had a similar effect on pDCs. CKm339 was slightly more effective (Figure 6).

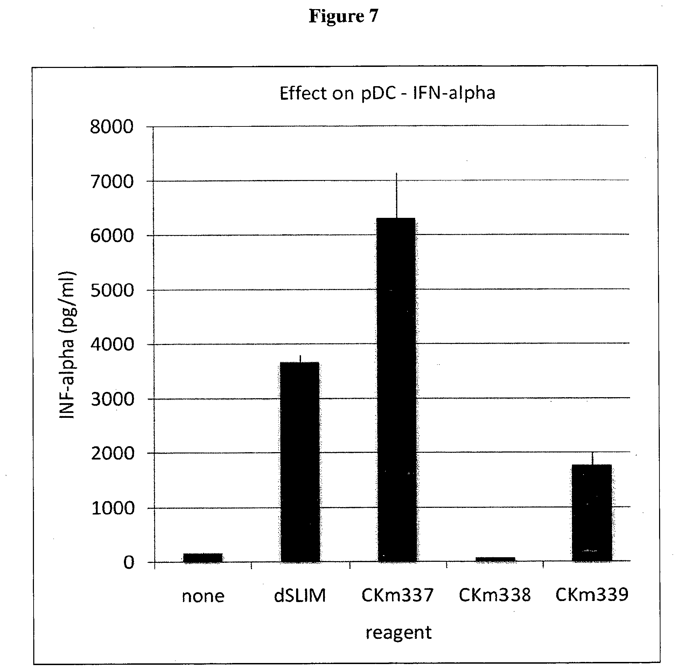

Of note, CKm337 had a surprisingly stronger effect on IFN-alpha secretion of pDCs compared with all other linear constructs (Figure 7).

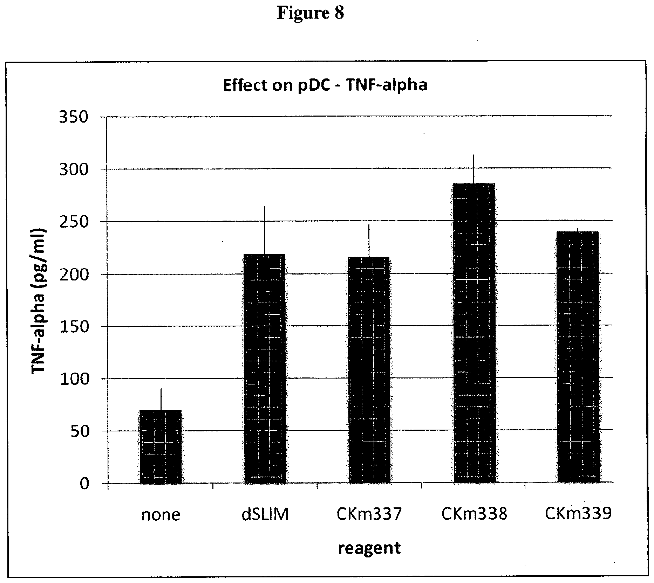

dSLIM, CKm337, CKm338 and CKm339 all had a similar effect on TNF-alpha secretion of pDCs.(Figure 8).

PBMCs were isolated from human buffy coats via a Ficoll density gradient. For functional analysis, 10⁶ cells/ml in medium (RPMI1640, 10% fetal calf serum, 100 Units/ml penicillin, 100 units/ml streptomycin 2 mM glutamine, 37°C, 5% CO₂) were stimulated for 2 days by the individual compounds applied at the indicated concentrations (2-3 µM).

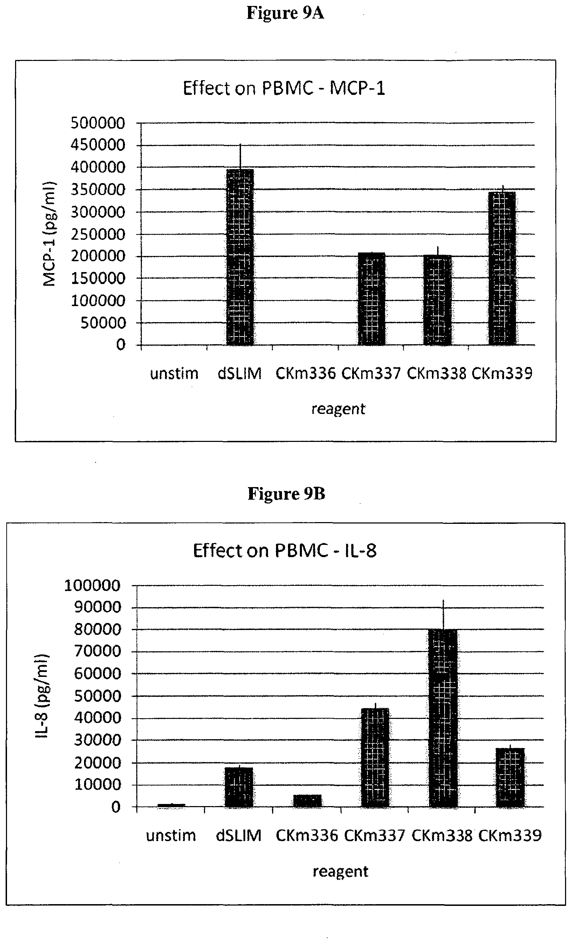

Figure 9 (A and B) shows the effect of the depicted DNA constructs (3 µM each) on PBMCs regarding secretion of MCP-1 and IL-8. As expected from the experiments with pDCs, the DNA construct with all nucleotides in L-conformation (CKm336) had no stimulatory capacity when applied to PBMCs. However, CKm337 was effective in provoking both MCP-1 and IL-8 secretion. Surprisingly, its effect concerning IL-8 secretion was stronger as compared to dSLIM and less strong concerning MCP-1 secretion.

For determination of cell subpopulations and activation status thereof, characteristic surface markers were labelled with selective fluorophore-conjugated antibodies. Antibody staining was performed with 10⁶ cells/staining set; each set was incubated with up to 4 different antibodies coupled to fluorophore-groups, finally resuspended in 400 µl FACS buffer and analysed by flow cytometry on at least 100,000 living cells. The gate strategy for determination of T cells and activated cells therein was CD3+/CD56- with the activation marker CD69.

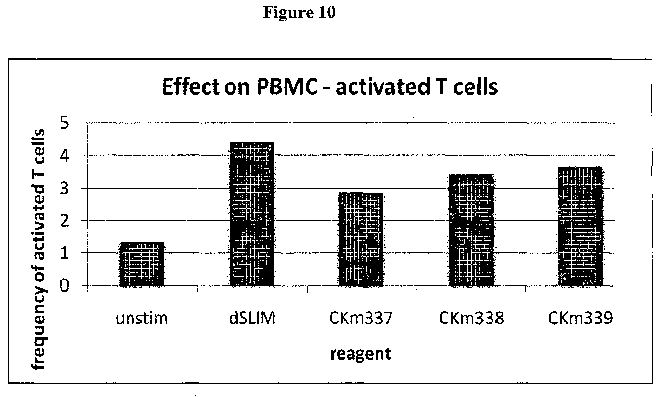

Figure 10 shows the effect of the depicted DNA constructs (2 µM each) on the frequency of activated T cells within the population of PBMCs. All five constructs had a comparable stimulatory capacity. T cells do not express TLR9. Therefore, upon stimulation with the DNA constructs cells within the PBMCs population were activated which in turn were capable of activating T cells.

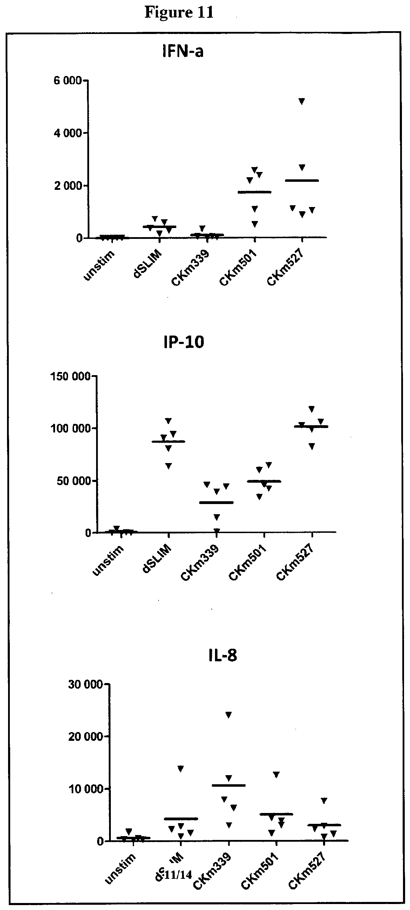

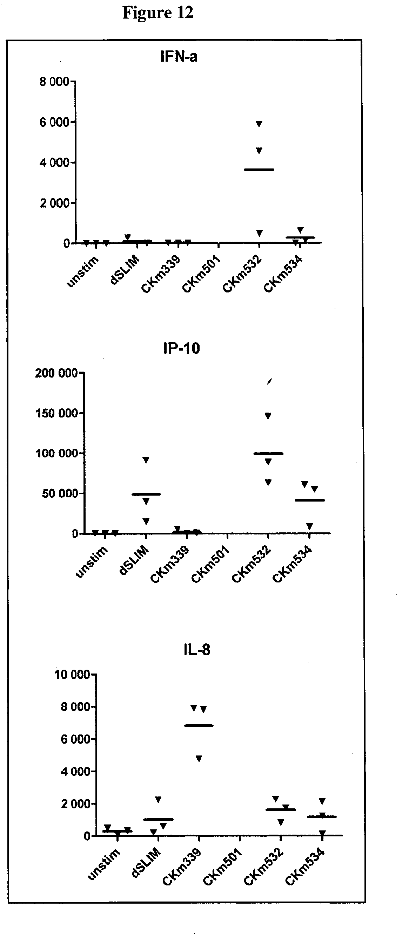

The optimisation of the sequences revealed that the introduction of G-stretches increases the efficacy of the oligonucleotides after transfection. The efficacy is further dependent on the distance between CG-motifs. The linear DNA sequences can be sufficiently protected against degradation by the use of L-ribose comprising deoxynucleotides at the 3' end of the oligonucleotide (comp table 2 and 3). The oligos CKm501 (SEQ ID NO: 33), CKm527 (SEQ ID NO: 32), CK 532 (SEQ ID NO: 31) and CKm534 (SEQ ID NO: 34) showed unexpected good results, as can be taken from table 3. Figures 11 and 12 show the effect of the indicated DNA constructs on the secretion of cytokines IFN-alpha (top), IP-10 (middle) and IL-8 (bottom) in PBMCs. The experiments were performed as already described above.

Figure 11 shows that CKm501 and CKm527 cause elevated levels of IFN-alpha secretion and CKm527 increases the IP-10 secretion too in comparison to dumbbell-shaped dSLIM. The secretion of IL-8 is comparable low with regard to dSLIM, but lower in comparison to CKm339, which is the sequence of single-stranded loops of dumbbell-shaped dSLIM protected on both ends with phosphorothioate modified deoxynucleotides.

As can be taken from figure 12 CKm532 shows a significant and unexpected high induction of IFN-alpha and IP-10 secretion, but a comparable low induction of IL-8 secretion. Thus, CKm532 confirms that the structural element of a G-stretch located directly at the 5' end and a further G-stretch located between two CG-motifs (second and third GC-motif) seems to be of advantage. Comparing CKm520 and CKm532 in Table 3 indicates that the location of a G-stretch between the second and third CG-motif in CKm532 is responsible for the intended increase in IFN-alpha and IP-10 secretion, whereas CKm520 mainly increases IL-8 secretion. Additionally the protection of the oligo only with two L-ribose comprising deoxynucleotides at the 3' end seems to be sufficient.

Shortening the G-stretch at the 5' end results in a reduction of efficacy as can be taken from the comparison of CKm532 and CKm 534 in Figure 12. Again, CKm532 demonstrates the advantages of the identified structural components with regard to an increased IFN-alpha and IP-10 secretion and a low IL-8 secretion.

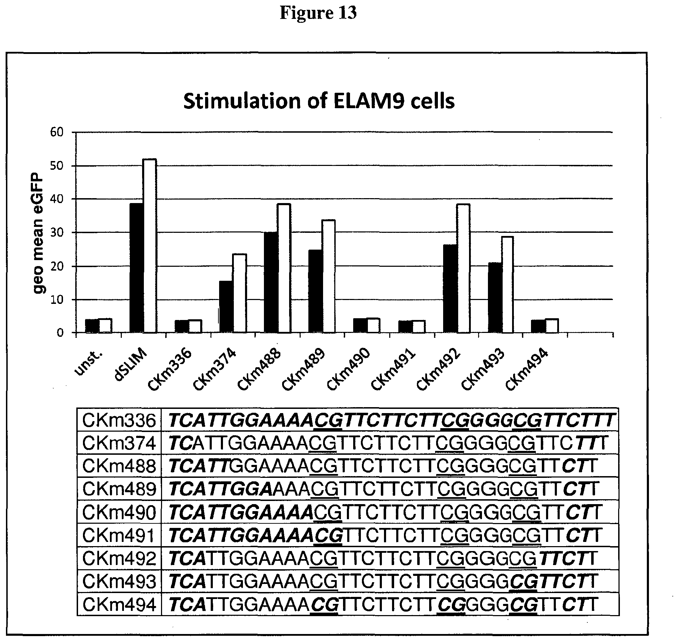

Figure 13 shows on top the results of ELAM9 cell stimulation with the indicated DNA constructs, which comprise deoxynucleotides with a different degree of L-ribose modifications. The L-ribose comprising nucleotides are represented in the sequences at the bottom of figure 13 in bold letters. The experiments were done in duplicate (L-dSLIM032 and L-dSLIM030).

The degree and position of L-ribose comprising deoxynucleotides has an influence on the stimulation of ELAM9 cells. A complete sequence in L-conformation (CKm 336; SEQ ID NO:2) does not have any stimulatory effect at all, which is in accordance with the disclosure of WO 2010/039137. Good effects are obtained by using CG-motif comprising oligos protected by L-ribose comprising deoxynucleotides at the 3' and 5' end, whereas a long extension of the L-ribose comprising deoxynucleotides at the 5'end is counterproductive (comp CKm489 and CKm490). Furthermore, the modification of CG-motifs with L-ribose comprising deoxynucleotides leads to a loss of effect. Thus, in order to achieve good stimulatory effects, the CG-motifs should not comprise L-riobose and the extension of L-ribose modified deoxynucleotides at both ends should be restricted, namely not more than eight terminal deoxynucleotides at the 5' and maximal the 3' terminal deoxynucleotides following the last CG-motif.

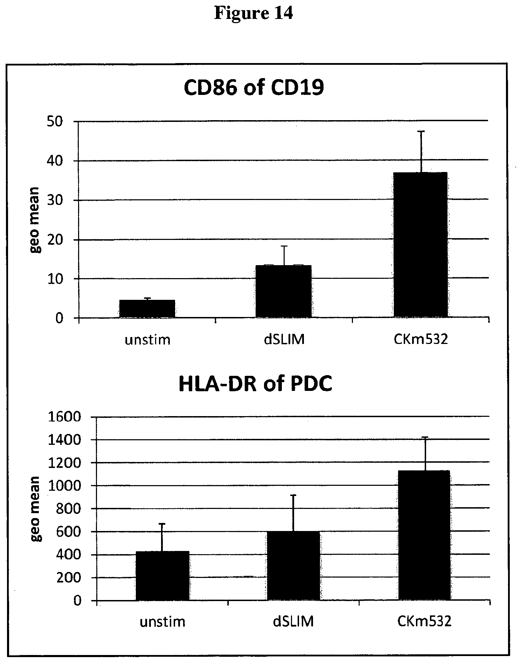

Figure 14 shows the immune stimulation by CKm532 and dSLIM, as compared to the unstimulated state. FACS experiments were performed according to the protocol employed for the experiments described in Figure 10 and adapted to B cells (gate Strategy: CD19 positive, CD86 as activation marker) and PDCs (gate strategy: lineage negative, HLA-DR positive, CD123 positive cells, CD40 and HLA-DR as activation marker), respectively. The data shown are based on measurements of three different buffy coat preparations.

The top of Figure 14 shows the stimulation of B cells, as evidenced by the marker CD86. Clearly, CKm532 causes an increased stimulation of B cells, when compared to dSLIM and the unstimulated state. This shows the increase in maturation of B cells, such as antibody-producing cells, which is an important feature of immune stimulation.

The bottom of Figure 14 shows the stimulation of PDCs, as detected using the marker HLA-DR. HLA-DR is part of the MHC molecules, and thus part of the antigen-presentation processes of the immune system. Again, CKm532 display a stronger increase of this immune stimulating feature, than dSLIM or the unstimulated cells.

In conclusion, CKm337 (D-DNA construct with nucleotides in L-conformation at both ends) surprisingly had a stimulatory effect on both PBMCs and isolated pDCs while the DNA construct with all nucleotides in L-conformation (CKm336) had no effect. Apparently, the conformation of CKm337 still allows binding to TLR9, and CKm336 is sterically incapable of binding to or stimulating TLR9.

Unexpectedly, the stimulation pattern induced by CKm337 in comparison to dumbbell-shaped dSLIM and phosphorothioat modified oligos, was unique compared to all other constructs. CKm337 induced the highest amounts of secreted IFN-alpha by pDCs. IL-8 secretion by PBMCs was weaker compared to phosphorothioate modified molecules, but stronger compared to dSLIM. In contrast, dSLIM induced a higher amount of secreted MCP-1 by PBMCs, but Ckm337 was comparable to the phosphorothioate-modified molecules.

It was possible to increase the effects observed with CKm337 by introducing so-called G-stretches directly at the 5' end of the linear DNA molecule. Additionally it turned out that the mere protection against degradation by L-ribose comprising deoxynucleotides at the 3' end is sufficient for stabilising the oligo. The identified structural features of G-stretch, CG-motifs, spacing of the CG-motifs and protection by using different degrees and positions of L-ribose modified deoxynucleotides allow a modulation of the immunostimulatory effect of L-ribose comprising oligonucleotides. It seems quite obvious, that the present disclosure reveals new tools for the construction of immunostimulatory DNA constructs for a targeted stimulation of cells or the immune system.

IFN-alpha has been known as an antiviral cytokine for many years. It stimulates Th1 cell development, therefore promoting the effects of CG-containing DNA molecules. IFN-alpha also exhibits antitumour activity in mouse and human malignancies and is capable of decreasing the tumourigenicity of transplanted tumour cells, partially by activating cytotoxic T cells and thereby increasing the likelihood of tumour-cell cytolysis. NK cell and macrophage activity, both also important for antitumour cytotoxicity, are also increased by IFN-alpha (Brassard et al., J. Leukoc. Biol. 2002 71: 565-81). Therefore, increasing the amount of IFN-alpha upon stimulation with the DNA constructs of the present disclosure is expected to be beneficial for the treatment of cancer.

IP-10 has been recently demonstrated to be a potent angiostatic protein in vivo. Thus, the induction of IP-10 especially in the treatment of tumour diseases seems to be of advantage too.

IL-8 is a proinflammatory cytokine, which is known to mediate the activation and migration of neutrophils into tissue from peripheral blood. The resulting neutrophilic infiltration may be partially responsible for inhibition of tumour growth as has been shown for ovarian cancer (Lee et al., J. Immunol. 2000 164: 2769-75). In addition, IL-8 is also chemotactic for T cells and basophils. Therefore, for treatment or prevention of at least some tumour types it is advantageous to selectively upregulate IL-8 in response to CG-containing DNA constructs. On the other hand it has been established that IL-8 triggers angiogenesis so that the induction of IL-8 secretion might be counterproductive. Thus, the differing degrees of IL-8 induction by the different DNA molecules of the present invention might allow for a tailoring of the molecule to the desired therapeutic effects.

MCP-1 is known to play a role in the recruitment of monocytes/macrophages to sites of injury and infection and is thereby possibly involved in stimulating host antitumour responses. It has been shown that MCP-1 can activate monocytes to be more cytostatic against several types of human tumour cells *in vitro* (Zachariae et al., J. Exp. Med. 1990 171: 2177-82). Therefore, similar to IL-8 it is beneficial to modulate MCP-1 expression depending on the specific tumour context.

Thus, the specific cytokine pattern induced is beneficial for treatment and prevention of distinct tumour types. Obviously, the specific context in which the unmethylated CG motif is presented to TLR9 determines the individual respective stimulation pattern induced in the responding cells.

## Claims

1. A non-coding, linear open-chained DNA construct for immunomodulation consisting of a sequence selected from the group consisting of SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 31, SEQ ID NO: 32, SEQ ID NO: 33, SEQ ID NO: 34 and SEQ ID NO: 35.

## Patentansprüche

1. Nicht codierendes lineares offen-kettiges DNA Konstrukt für die Immunmodulation bestehend aus einer Sequenz ausgewählt aus der Gruppe bestehend aus SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 31, SEQ ID NO: 32, SEQ ID NO: 33, SEQ ID NO: 34 und SEQ ID NO: 35.

## Revendications

1. Construction d'ADN à chaîne ouverte linéaire, non codant pour une immunomodulation constituée d'une séquence choisie dans le groupe constitué par SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 31, SEQ ID NO: 32, SEQ ID NO: 33, SEQ ID NO: 34 et SEQ ID NO: 35.
